# EUROPEAN PATENT APPLICATION

(11) **EP 4 843 475 A1**
(43) Date of publication of application: **25.11.2026**
(21) Application number: 25741906.9
(22) Date of filing: 10.01.2025
(51) Int. Cl.: A61B 1/045, A61B 1/00

(54) **IMAGE PROCESSING METHOD, IMAGE PROCESSING DEVICE, IMAGE PROCESSING PROGRAM, AND ENDOSCOPE SYSTEM**

(30) Priority: 18.01.2024 JP 2024006308
(71) Applicant: FUJIKURA LTD., Tokyo 135-8512 (JP); Fibertech Co., Ltd., Sakura-shi, Chiba, 285-0074 (JP)
(72) Inventor: NAKATATE, Kenichi, Sakura-shi, Chiba 285-8550 (JP); MATSUO, Shoichiro, Sakura-shi, Chiba 285-8550 (JP); KAMEYAMA, Takashi, Sakura-shi, Chiba 285-8550 (JP); YAMADA, Koji, Sakura-shi, Chiba 285-0074 (JP); NIHONYANAGI, Kazuyuki, Sakura-shi, Chiba 285-0074 (JP); HIRATA, Norifumi, Gotenba-shi, Shizuoka 412-0047 (JP); KAWAI, Hideki, Gotenba-shi, Shizuoka 412-0047 (JP); MAKI, Jiro, Gotenba-shi, Shizuoka 412-0047 (JP)
(74) Representative: Plasseraud IP
(86) International application number: PCT/JP2025/000697
(87) International publication number: WO 2025/154680

(57) **Abstract**

Provided is an image processing technology that improves resolution or visibility of an obtained image. An image processing device (10) acquires a plurality of target images obtained by capturing an image of an end of an image guide (13q), which has a plurality of cores and the other end of which faces a target object (O), and generates a super-resolution image by performing simple averaging or weighted averaging of pixel values of pixels corresponding to a same point of the target object (O) in the plurality of target images.

## Description

### Technical Field

The present invention relates to an image processing method, an image processing device, and an image processing program, each of which is for processing an image captured with use of an image guide. The present invention also relates to an endoscope system including such an image processing device.

### Background Art

An endoscope system is widely used which obtains an image including, as a subject, a target object inside a body by imaging one end of an image guide that has the other end inserted into the body. For example, Patent Literature 1 discloses an angioscope system in which a risk of damage to an intima of a small-diameter blood vessel is mitigated by reducing a diameter and increasing flexibility of an image guide.

### Citation List

### [Patent Literature]

[Patent Literature 1]
Japanese Patent Application Publication Tokukaihei No. 8-191439

### Summary of Invention

### Technical Problem

In an endoscope system using an image guide, resolution is determined by a core density of the image guide. For example, a structure having a size of approximately 100 times a core-to-core distance can be visually recognized from an image that is formed on an end face of the image guide. However, it becomes difficult to visually recognize, from the image formed on the end face of the image guide, a structure having a size of approximately 10 times the core-to-core distance. Further, it becomes impossible to visually recognize, from the image formed on the end face of the image guide, a structure having a size comparable to the core-to-core distance.

In the angioscope system disclosed in Patent Literature 1, image quality of an obtained image is improved by correcting a difference in brightness and color tone between pixels which is caused by a difference in transmission characteristic between cores of the image guide. However, such correction cannot lead to resolution higher than the resolution that is determined by the core density of the image guide.

One aspect of the present invention has been made in view of the above problem, and an object of an aspect of the present invention is to provide an image processing technology that improves resolution or visibility of an obtained image.

### Solution to Problem

An image processing method in accordance with an aspect of the present invention includes: an acquisition process of acquiring a plurality of target images obtained by capturing an image of an end of an image guide, which has a plurality of cores and the other end of which faces a target object; and a super-resolution process of generating a super-resolution image by performing simple averaging or weighted averaging of pixel values of pixels corresponding to a same point of the target object in the plurality of target images.

An image processing device in accordance with an aspect of the present invention includes at least one processor, the processor being configured to execute: an acquisition process of acquiring a plurality of target images obtained by capturing an image of an end of an image guide, which has a plurality of cores and the other end of which faces a target object; and a super-resolution process of generating a super-resolution image by performing averaging or weighted averaging of pixel values of pixels corresponding to a same point of the target object in the plurality of target images.

An image processing program in accordance with an aspect of the present invention is for causing at least one processor to operate, the program causing the processor to execute: an acquisition process of acquiring a plurality of target images obtained by capturing an image of an end of an image guide, which has a plurality of cores and the other end of which faces a target object; and a super-resolution process of generating a super-resolution image by performing averaging or weighted averaging of pixel values of pixels corresponding to a same point of the target object in the plurality of target images.

### Advantageous Effects of Invention

One aspect of the present invention can improve resolution or visibility of an obtained image.

### Brief Description of Drawings

Fig. 1 is a schematic diagram illustrating a configuration of an endoscope system in accordance with an embodiment of the present invention.
Fig. 2 is a block diagram illustrating a configuration of an image processing device included in the endoscope system illustrated in Fig. 1.
Fig. 3 is a flow chart showing a flow of an image processing method in accordance with an embodiment of the present invention.
Fig. 4 is a diagram showing an execution example of the image processing method illustrated in Fig. 3. Specifically, Fig. 4 shows a target object to be observed in the execution example.
Fig. 5 is a diagram showing an execution example of the image processing method illustrated in Fig. 3. Specifically, Fig. 5 shows a mesh image which is obtained by a white standard plate imaging process in the execution example.
Fig. 6 is a diagram showing an execution example of the image processing method illustrated in Fig. 3. Specifically, Fig. 6 shows a first target image which is obtained by the target object imaging process in the execution example.
Fig. 7 is a diagram showing an execution example of the image processing method shown in Fig. 3. Specifically, Fig. 7 shows a second target image which is obtained by the target object imaging process in the execution example.
Fig. 8 is a diagram showing an execution example of the image processing method shown in Fig. 3. Specifically, Fig. 8 shows a third target image which is obtained by the target object imaging process in the execution example.
Fig. 9 is a diagram showing an execution example of the image processing method shown in Fig. 3. Specifically, Fig. 9 shows a fourth target image which is obtained by the target object imaging process in the execution example.
Fig. 10 is a diagram showing an execution example of the image processing method shown in Fig. 3. Specifically, Fig. 10 shows a super-resolution image which is obtained by a super-resolution process (simple averaging) in the execution example.
Fig. 11 is a diagram showing an execution example of the image processing method shown in Fig. 3. Specifically, Fig. 11 shows an output image which is obtained by a mask process in the execution example.
Fig. 12 is a flow chart showing a flow of an image processing method in accordance with a variation of the image processing method shown in Fig. 3.
Fig. 13 is a diagram showing an execution example of the image processing method shown in Fig. 12. Specifically, Fig. 13 shows a first target image which is obtained by a transparency process in the execution example.
Fig. 14 is a diagram showing an execution example of the image processing method shown in Fig. 12. Specifically, Fig. 14 shows a second target image which is obtained by the transparency process in the execution example.
Fig. 15 is a diagram showing an execution example of the image processing method shown in Fig. 12. Specifically, Fig. 15 shows a third target image which is obtained by the transparency process in the execution example.
Fig. 16 is a diagram showing an execution example of the image processing method shown in Fig. 12. Specifically, Fig. 16 shows a fourth target image which is obtained by the transparency process in the execution example.
Fig. 17 is a diagram showing an execution example of the image processing method shown in Fig. 12. Specifically, Fig. 17 shows a super-resolution image which is obtained by a super-resolution process (weighted averaging) in the execution example.
Fig. 18 is a diagram showing an execution example of the image processing method shown in Fig. 12. Specifically, Fig. 18 shows an output image which is obtained by a mask process in the execution example.

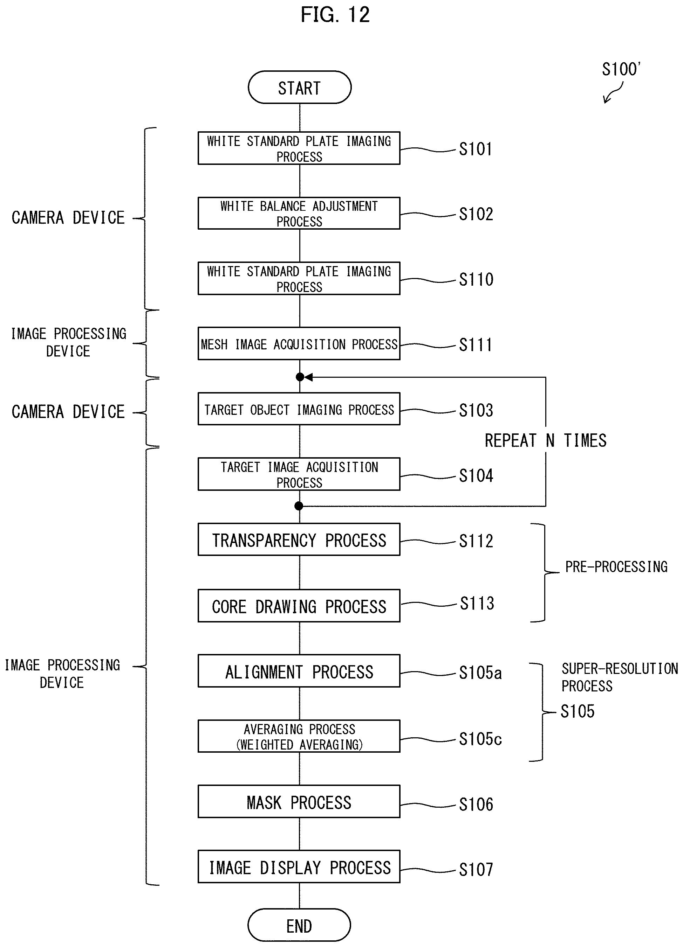

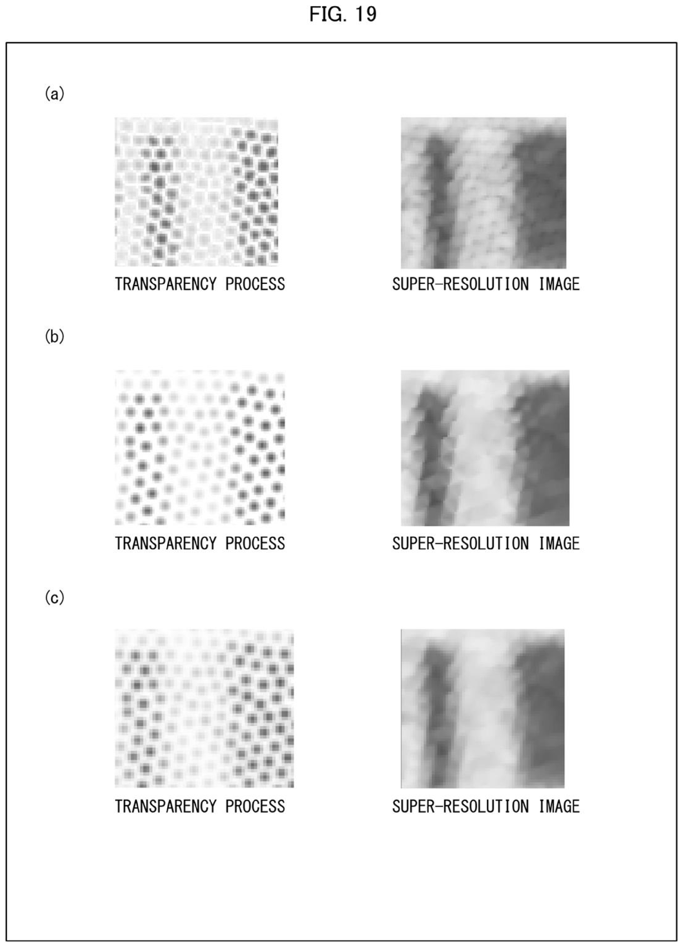

Fig. 19 is a diagram showing an execution example of the image processing method shown in Fig. 12. Specifically, Fig. 19 is an enlarged view showing a part of a super-resolution image which is obtained by a core drawing process in the execution example. (a) of Fig. 19 shows a case where opacity is not adjusted. (b) of Fig. 19 shows a case where the opacity of each pixel having a distance from the center of a core region equal to or less than a predetermined threshold is adjusted so as to decrease linearly as the distance increases. (c) of Fig. 19 corresponds to a case where the opacity of each pixel having a distance from the center of the core region equal to or less than the predetermined threshold is adjusted so as to decrease according to a Gaussian distribution as the distance increases.

### Description of Embodiments

### (Configuration of endoscope system)

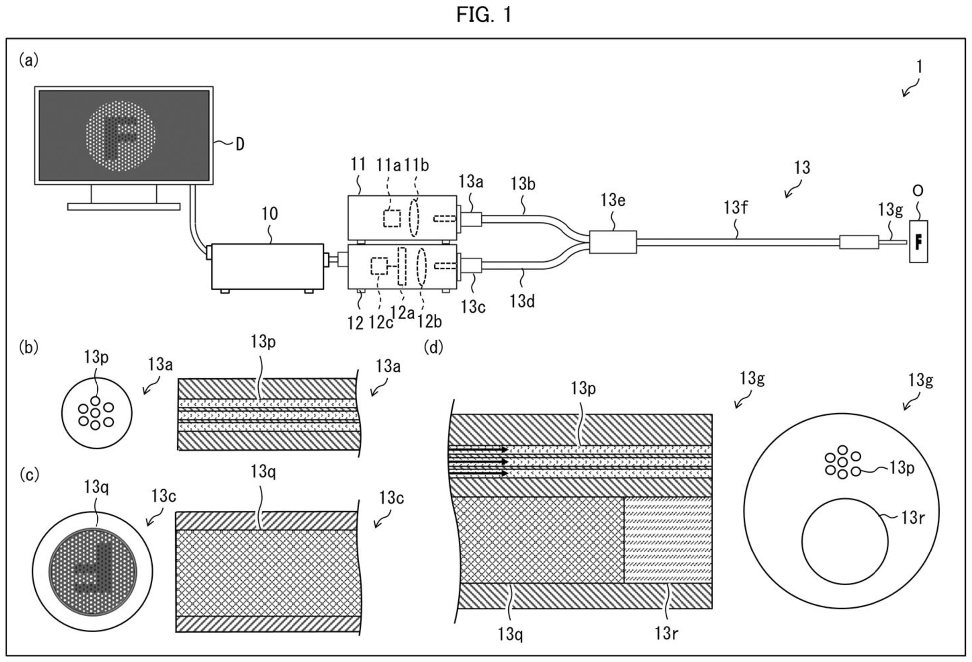

The following description will discuss an endoscope system 1 in accordance with an embodiment of the present invention, with reference to Fig. 1. In Fig. 1, (a) is a schematic diagram illustrating a configuration of the endoscope system 1, (b) shows a plan view and a cross-sectional view illustrating a structure of a light guide 13p in a light guide plug part 13a, (c) shows a plan view and a cross-sectional view illustrating a structure of an image guide 13q in an image guide plug part 13c, and (d) shows a plan view and a cross-sectional view illustrating structures of the light guide 13p and the image guide 13q in an insertion part 13g.

The endoscope system 1 is for displaying, on a display D, an image of a target object O to be observed. As shown in (a) of Fig. 1, the endoscope system 1 includes an image processing device 10, a light source device 11, a camera device 12, and an endoscope probe 13.

As shown in (a) of Fig. 1, the light source device 11 includes a light source 11a and a condenser lens 11b. The light source 11a is disposed on an optical axis of the condenser lens 11b. As the light source 11a, for example, a light emitting diode (LED), a xenon lamp, a halogen lamp, or the like is used.

As shown in (a) of Fig. 1, the camera device 12 includes an image sensor 12a, an objective lens 12b, and a signal processing circuit 12c. The image sensor 12a is disposed on an optical axis of the objective lens 12b. The signal processing circuit 12c is electrically connected to the image sensor 12a. As the image sensor 12a, for example, a complementary metal oxide semiconductor (CMOS) sensor, a charge coupled device (CCD) sensor, or the like is used.

As shown in (a) of Fig. 1, the endoscope probe 13 includes: (1) the light guide plug part 13a that is mechanically connected to the light source device 11; (2) a light guide lead part 13b that has one end connected to the light guide plug part 13a; (3) an image guide plug part 13c that is mechanically connected to the camera device 12; (4) an image guide lead part 13d that has one end connected to the image guide plug part 13c; (5) a branching part 13e that has one end connected to the other ends of the light guide lead part 13b and the image guide lead part 13d; (6) an intermediate part 13f that has one end connected to the other end of the branching part 13e; and (7) an insertion part 13g that has one end connected to the other end of the intermediate part 13f. Note that the endoscope probe 13 may further include a water supply lumen, a working lumen, an insertion part bending mechanism, and a bending operation part (all not illustrated).

As illustrated in (b) and (d) of Fig. 1, one or a plurality of light guides 13p that function as waveguides for guiding illumination light which is generated by the light source device 11 are provided inside the light guide plug part 13a, the light guide lead part 13b, the branching part 13e, the intermediate part 13f, and the insertion part 13g. Each of the light guides 13p is embedded in a cladding. The light guides 13p that are included in the light guide plug part 13a and the light guide lead part 13b are bundled with the image guide 13q (described later) in the branching part 13e, and are enclosed in the intermediate part 13f and the insertion part 13g together with the image guide 13q.

Further, as illustrated in (c) and (d) of Fig. 1, the image guide 13q, which is a waveguide for guiding scattered light generated at the target object O, is provided inside the image guide plug part 13c, the image guide lead part 13d, the branching part 13e, the intermediate part 13f, and the insertion part 13g. The image guide 13q is composed of a cladding and a plurality of cores (for example, several thousand to several tens of thousands of cores) which are regularly or randomly arranged inside the cladding. The image guide 13q that is included in the image guide plug part 13c and the image guide lead part 13d is bundled with the light guide 13p described above in the branching part 13e, and is enclosed in the intermediate part 13f and the insertion part 13g together with the light guide 13p.

As the image guide 13q, for example, an image guide fiber, an image fiber, a fiber bundle, a fiber conduit, or the like can be used. A material of the image guide 13q may be any of a quartz-based material, a multi-component glass-based material, and a plastic-based material. Further, the number of cores and core arrangement of the image guide 13q are not particularly limited. As an example, an image guide fiber in which 3000 cores are arranged on lattice points of a hexagonal close-packed lattice can be used as the image guide 13q. Note, however, that the image guide 13q has a lower resolution than the image sensor 12a, that is, the number of cores of the image guide 13q is smaller than the number of cells of the image sensor 12a.

Further, as shown in (d) of Fig. 1, an objective lens 13r is provided at a distal end of the insertion part 13g. One end of the objective lens 13r constitutes an end face of the insertion part 13g, and the other end of the objective lens portion 13s faces one end of the image guide 13q. As the objective lens 13r, for example, a GRIN lens or a lens unit composed of a plurality of lenses can be used.

The endoscope system 1 operates, for example, as follows. That is, illumination light is emitted from the light source 11a in a state in which the insertion part 13g is inserted into a body of a patient. The illumination light emitted from the light source 11a is condensed on an end face of the light guide 13p on the light guide plug part 13a side by the condenser lens 11b. The illumination light that has entered cores of the light guide 13p through the end face of the light guide 13p on the light guide plug part 13a side is guided through the cores of the light guide 13p. Then, the illumination light is emitted from the cores of the light guide 13p through an end face of the light guide 13p on the insertion part 13g side. The illumination light emitted from the cores of the light guide 13p through the end face of the light guide 13p on the insertion part 13g side illuminates the target object O inside the body of the patient.

Scattered light which has been generated at points of the target object O by scattering the illumination light inside the body of the patient is condensed at respective corresponding points of the end face of the image guide 13q on the insertion part 13g side by the objective lens 13r. As a result, an image of the target object O is formed on the end face of the image guide 13q on the insertion part 13g side. The scattered light that has entered the cores of the image guide 13q through the end face of the image guide 13q on the insertion part 13g side via the objective lens 13r is guided through the cores of the image guide 13q. Then, the scattered light exits from the cores of the image guide 13q through an end face of the image guide 13q on the image guide plug part 13c side. As a result, the image of the target object O is also formed on the end face of the image guide 13q on the image guide plug part 13c side, similarly to the end face of the image guide 13q on the insertion part 13g side.

Light having exited from the cores of the image guide 13q through the end face of the image guide 13q on the image guide plug part 13c side (light derived from the scattered light that has been generated at the points of the target object O) is condensed at respective corresponding points of a light receiving surface of the image sensor 12a by the objective lens 12b. As a result, an image of the target object O is formed on the light receiving surface of the image sensor 12a. The image sensor 12a generates an electric signal representing the image of the target object O, and inputs, to the signal processing circuit 12c, the electric signal thus generated. The signal processing circuit 12c generates a target image representing the image of the target object O from the electric signal which has been inputted from the image sensor 12a.

The camera device 12 inputs, to the image processing device 10, the target image thus generated. The image processing device 10 generates an output image to be displayed on the display D, from the target image which has been inputted from the camera device 12, according to an image processing method S100 (described later). Note that a function performed by the image processing device 10 may be performed by the camera device 12. That is, a configuration in which the camera device 12 performs the image processing method S100 (described later) may be adopted instead of a configuration in which the image processing device 10 performs the image processing method S100. In this case, the image processing device 10 can be omitted from the endoscope system 1.

Note that in the present specification, the "image" refers to a two-dimensional array of pixel values. A pixel value of a monochrome image consists of, for example, one numerical value corresponding to brightness. In a D-gradation monochrome image, the brightness takes an integer value of 0 or more and D-1 or less. A pixel value in a color image consists of, for example, three numerical values corresponding to RGB. In this case, R represents a red component, G represents a green component, and B represents a blue component. In a D-gradation color image, the red component, the green component, and the blue component each take an integer value of 0 or more and D-1 or less. A pixel value in a transparent color image consists of, for example, four numerical values corresponding to RGBA. In this case, A represents opacity. In the case of a D-gradation transparent color image, the red component, the green component, the blue component, and the opacity each take an integer value of 0 or more and D-1 or less.

A pixel in an image is identified by coordinates (x, y). In a case where the image has a size of W pixels in width and H pixels in length, x is an integer of 0 or more and W-1 or less and y is an integer of 0 or more and H-1 or less. A pixel having the coordinates (x, y) is hereinafter also referred to as a pixel (x, y). For example, a pixel (0, 0) is at an upper left corner of an image, a pixel (1, 0) is immediately to the right of the pixel (0, 0), and a pixel (0, 1) is immediately below the pixel (0, 0). The pixel value (brightness value) of a pixel (x, y) of a monochrome image is denoted by A(x, y). Further, regarding the pixel values of a pixel (x, y) of a color image, a red component of the pixel is denoted by R(x, y), a green component of the pixel is denoted by G(x, y), and a blue component of the pixel is described by B(x, y). Similarly, regarding the pixel value of a pixel (x, y) of a color image having opacity, a red component of the pixel is denoted by R(x, y), a green component of the pixel is denoted by G(x, y), a blue component of the pixel is denoted by B(x, y), and the opacity of the pixel is denoted by A(x, y).

A file format for saving an image is not particularly limited. For example, in a case where a file format such as PNG, JPEG, or GIF is used, it is possible to save a monochrome image or a color image. Further, in a case where a file format such as transparent PNG is used, it is possible to save a transparent color image.

### (Configuration of image processing device)

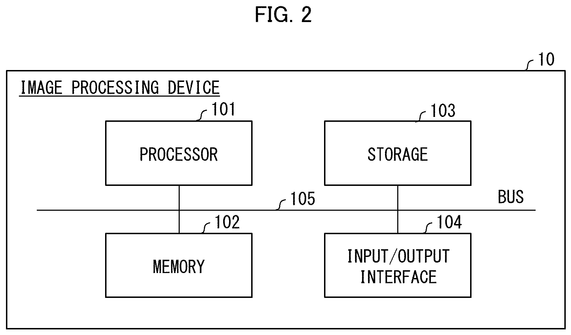

The following description will discuss a configuration of the image processing device 10, with reference to Fig. 2. Fig. 2 is a block diagram illustrating a configuration of the image processing device 10.

As illustrated in Fig. 2, the image processing device 10 includes a processor 101, a memory 102, a storage 103, an input/output interface 104, and a bus 105. The processor 101, the memory 102, the storage 103, and the input/output interface 104 are connected to each other via the bus 105.

The memory 102 is a configuration for loading and storing an image processing program P100 for executing the image processing method S100 (described later), in a manner that the processor 101 can refer to the image processing program P100. Further, the memory 102 is also used for storing various images which are acquired from the camera device 12. Note that as the memory 102, for example, a semiconductor random access memory (RAM) or the like can be used.

The processor 101 is configured to execute the image processing method S100 (described later) according to the image processing program P100 which has been loaded on the memory 102. As the processor 101, for example, a central processing unit (CPU), a graphic processing unit (GPU), or a combination thereof can be used.

The storage 103 is a configuration for storing (nonvolatile storage) the image processing program P100. When executing the image processing method S100 (described later), the processor 101 loads the image processing program P100 stored in the storage 103 onto the memory 102 and refers to the image processing program P100. Note that, as the storage 103, for example, a flash memory, a hard disk drive (HDD), a solid state drive (SSD), or any combination thereof can be used.

The input/output interface 104 is configured to connect each of the camera device 12 and the display D to the image processing device 10. Note that as the input/output interface 104, for example, a universal serial bus (USB) interface or the like can be used.

Note that the above description has discussed a configuration in which the image processing method S100 (described later) is executed by the single processor 101 provided in a single computer (a computer that functions as the image processing device 10), but this should not be construed as a limitation. That is, it is also possible to employ a configuration in which a plurality of processors gathered in a single computer or provided dispersedly in a plurality of computers work together to execute the image processing method S100 (described later).

The image processing program P100 may be stored in a computer-readable non-transitory tangible recording medium. The recording medium may be the memory 102, the storage 103, or another recording medium. For example, a tape, a disk, a card, a semiconductor memory, a programmable logic circuit, and the like can be used as another recording medium.

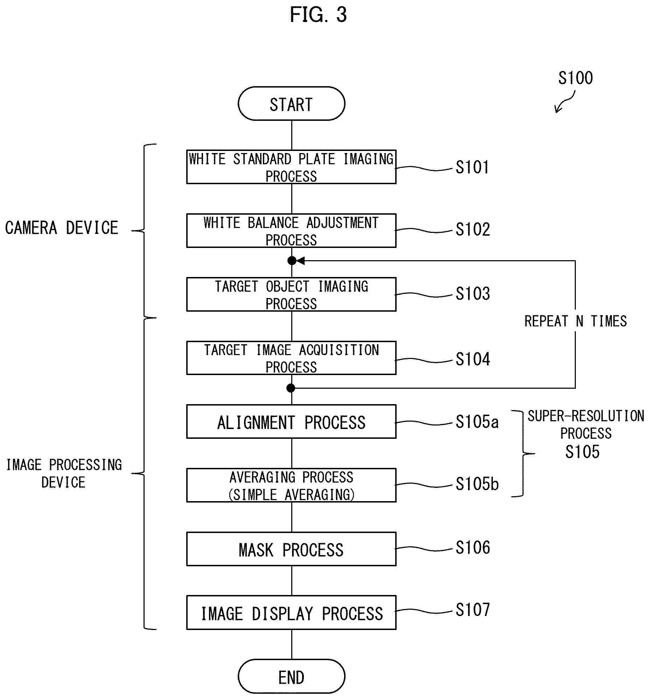

(Flow of image processing method) The following description will discuss a flow of the image processing method S100 in accordance with an embodiment of the present invention, with reference to Fig. 3. Fig. 3 is a flowchart showing the flow of the image processing method S100.

As illustrated in Fig. 3, the image processing method S100 includes a white standard plate imaging process S101, a white balance adjustment process S102, a target object imaging process S103, a target image acquisition process S104, a super-resolution process S105, a mask process S106, and an image display process S107. The white standard plate imaging process S101, the white balance adjustment process S102, and the target object imaging process S103 are each a process which is primarily performed by the camera device 12. On the other hand, the target image acquisition process S104, the super-resolution process S105, the mask process S106, and the image display process S107 are each a process which is primarily performed by the image processing device 10.

The white standard plate imaging process S101 is a process in which a user causes the objective lens 13r of the endoscope probe 13 to face a white standard plate, and the camera device 12 captures an image of the end face of the image guide 13q on the image guide plug part 13c side. The white balance adjustment process S102 is a process in which the camera device 12 adjusts white balance with reference to the image captured in the white standard plate imaging process S101. The image that is captured in the white standard plate imaging process S101 and that is referred to in the white balance adjustment process S102 is a color image in which a region corresponding to the cores of the image guide 13q is bright and other regions are dark. This image is hereinafter referred to as a mesh image I0. Note that a specific example of the mesh image I0 which is obtained in the white standard plate imaging process S101 will be described later with reference to a different drawing.

The target object imaging process S103 is a process in which a user causes the objective lens 13r of the endoscope probe 13 to face the target object O, and the camera device 12 captures an image of the end face of the image guide 13q on the image guide plug part 13c side. The target image acquisition process S104 is a process in which the image processing device 10 acquires, from the camera device 12, the image captured in the target object imaging process S103, and stores the image in the memory 102. The target object imaging process S103 and the target image acquisition process S104 are repeated n times (n is a predetermined natural number). The image which is captured in the target object imaging process S103 and acquired in the target image acquisition process S104 is a color image which represents an image of the target object O formed on the end face of the image guide 13q on the image guide plug part 13c side. Such images are hereinafter referred to as target images I1, I2, ... , In. Note that specific examples of the target images I1, I2, ... , In which are obtained in the target object imaging process S103 will be described later with reference to different drawings.

The super-resolution process S105 is a process in which a super-resolution image I is generated by averaging pixel values of pixels corresponding to the same point of the target object O in the target images I1, I2, ... , In. In the present embodiment, the super-resolution process S105 includes an alignment process S105a and an averaging process S105b.

The alignment process S105a is a process in which, for each target image Ii (i is each natural number of not less than 1 and not greater than n-1) other than the target image In which is acquired last, the image processing device 10 (1) calculates a displacement vector vi of the target image Ii relative to the target image In, and (2) shifts the pixel value of each pixel of the target image Ii by the displacement vector vi. As a method of calculating the displacement vector vi, for example, a known method using feature amounts of the target images Ii and In can be used. An image which is obtained by the alignment process S105a for the target image Ii is a color image which represents an image of the target object O formed on the end face of the image guide 13q on the image guide plug part 13c side. This image is hereinafter referred to as a target image Ii'.

When a pixel (xi, yi) of the target image Ii and a pixel (xn, yn) of the target image In correspond to the same point of a subject, the displacement vector vi = (vix, viy) is given, for example, by the following formulas.$vix = xi - xn ,$ and $viy = yi - yn .$

Further, in a case where a pixel value at a pixel (x, y) of the target image Ii is given by {Ri(x, y), Gi(x, y), Bi(x, y)}, a pixel value {Ri'(x, y), Gi'(x, y), Bi'(x, y)} at a pixel (x, y) of the target image Ii' is given, for example, by the following formulas:
if $0 \leq x + vix \leq W and 0 \leq y + viy \leq H ,$ $Ri ′ \left(x, y\right) = Ri \left(x+vix, y+viy\right) ,$ $Gi ′ \left(x, y\right) = Gi \left(x+vix, y+viy\right) ,$ and $Bi ′ \left(x, y\right) = Bi \left(x+vix, y+viy\right) ;$ and
if $x + vix < 0 , W < x + vix , y + viy < 0 or H < y + viy ,$ $Ri ′ \left(x, y\right) = 0 ,$ $Gi ′ \left(x, y\right) = 0 ,$ and $Bi ′ \left(x, y\right) = 0 .$

Note that the alignment process S105a is not performed for the target image In which is acquired last. However, for convenience of description, the target image In is hereinafter also referred to as a target image In'. That is, a pixel value {Rn'(x, y), Gn'(x, y), Bn'(x, y)} at a pixel (x, y) of the target image In' is the same as a pixel value {Rn(x, y), Gn(x, y), Bn(x, y)} at a pixel (x, y) of the target image In. Specific examples of the target images I1', I2', ... , In' which are obtained by the alignment process S105a will be described later with reference to different drawings.

Note that the above description has discussed a configuration in which, by using, as a reference image, the target image In which is acquired last, respective pixel values of the target images Ii other than the reference image are shifted, but this should not be construed as a limitation. That is, it is also possible to employ a configuration in which, by using, as a reference value, a target image Ip (p is a predetermined natural number of not less than 1 and not greater than n-1) other than the target image In which is acquired last, respective pixel values of target images Iq (q is different from p and each natural number of not less than 1 and not greater than n) other than the reference image are shifted.

The averaging process S105b is a process in which, for each pixel (x, y), the image processing device 10 performs simple averaging for each component of the pixel values {Rj'(x, y), Gj'(x, y), Bj'(x, y)} of respective target images Ij' (j is each natural number of 1 or more and n or less), that is, simple averaging of the pixel values Rj'(x, y) of respective target images Ij', simple averaging of pixel values Gj'(x, y) of respective target images Ij', and simple averaging of pixel values Bj'(x, y) of respective target images Ij'. An image which is obtained by the averaging process S105b is a color image which represents an image of the target object O formed on the end face of the image guide 13q on the image guide plug part 13c side. This image is hereinafter referred to as the super-resolution image I. A pixel value {R(x, y), G(x, y), B(x, y)} at a pixel (x, y) of the super-resolution image I is given, for example, by the following formulas. A specific example of the super-resolution image I generated in the super-resolution process S105 will be described later with reference to different drawings.$R \left(x, y\right) = \left\{R1′\left(x, y\right)+R2′\left(x, y\right)+…+Rn′\left(x, y\right)\right\} / n ,$ $G \left(x, y\right) = \left\{G1′\left(x, y\right)+G2′\left(x, y\right)+…+Gn′\left(x, y\right)\right\} / n ,$ $B \left(x, y\right) = \left\{B1′\left(x, y\right)+B2′\left(x, y\right)+…+Bn′\left(x, y\right)\right\} / n .$

The mask process S106 is a process in which the image processing device 10 generates an output image I' by masking a region outside a view circle in the super-resolution image I. Here, masking a region outside the view circle refers, for example, to setting, in the super-resolution image I, a pixel value {R(x, y), G(x, y), B(x, y)} of a pixel (x, y) located outside the view circle to R(x, y) = 0, G(x, y) = 0, and B(x, y) = 0. A specific example of the output image I' generated in the mask process S106 will be described later with reference to different drawings.

The image display process S107 is a process in which the image processing device 10 displays the output image I' on the display D. Hereby, a series of processes of the image processing method S100 is completed.

Note that the above description has discussed the image processing method S100 for displaying a still image of the target object O on the display D. This method may be used to display a moving image of the target object O on the display D. In such a case, the camera device 12 repeatedly performs the target object imaging process S103 at a cycle corresponding to a frame rate. Further, the image processing device 10 performs the super-resolution process S105, the mask process S106, and the image display process S107 on latest n target images among target images captured by the camera device 12 up to that time point. Thus, the moving image of the target object O can be displayed on the display D.

Note that as a pre-process of the super-resolution process S105, deformation correction such as affine transformation may be performed on each of the target images I1, I2, ..., In. In a case where a difference occurs in positions and shapes of images of cores included in the target images I1, I2, ... , In due to a difference in tilt angles at the time of imaging, this difference is eliminated in advance by the deformation correction. As a result, it is possible to obtain a super-resolution image I that has higher visibility. Further, as a pre-process of the super-resolution process S105, color unevenness correction and brightness unevenness correction, which are disclosed in Japanese Patent Application Publication, Tokukai, No. 2021-177604 A (Japanese Patent No. 7189173) may be performed on each of the target images I1, I2, ... , In. In a case where color unevenness and brightness unevenness occur in each image of the cores included in the target images I1, I2, ... , In, the color unevenness and the brightness unevenness are eliminated in advance by the color unevenness correction and the brightness unevenness correction. As a result, it is possible to obtain a super-resolution image I that has higher visibility.

### (Execution example of image processing method)

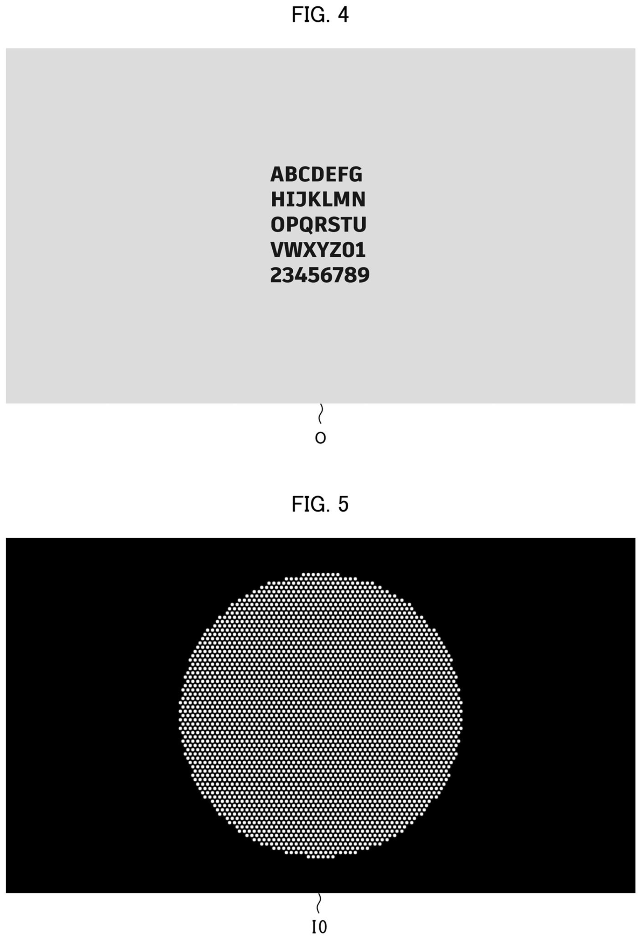

The following will discuss an execution example of the image processing method S100 with reference to Figs. 4 to 11. In this specific example, for simplicity, the number n of target images I1, I2, ... , In is set to 4.

Fig. 4 is a plan view of a target object O which is used as a sample in this execution example. The target object O which is used as a sample in this execution example is a white standard plate on which alphabets from A to Z and numbers from 0 to 9 are printed.

Fig. 5 is a diagram showing, by way of example, the mesh image I0 which is obtained in the white standard plate imaging process S101. As described above, the mesh image I0 is an image in which a region corresponding to the cores of the image guide 13q is bright and other regions are dark.

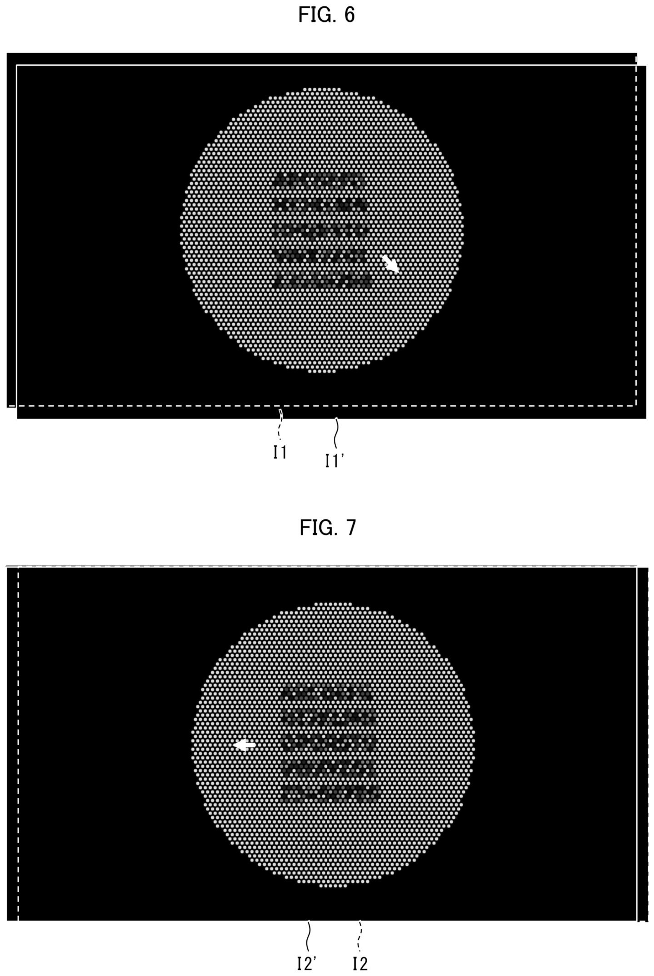

Fig. 6 is a diagram showing, by way of example, the target image I1 which is obtained in the target object imaging process S103 and the target image I1' which is obtained in the alignment process S105a. Positions of the characters and alphabets which are included as subjects in the target image I1 are shifted to the lower right relative to positions of the characters and alphabets which are included as subjects in the target image I4 that is used as a reference image. Therefore, a displacement vector v1 points to the lower right as indicated by a white arrow in Fig. 6. Positions of the characters and alphabets which are included as subjects in the target image I1' which is obtained by shifting a pixel value of each pixel of the target image I1 by the displacement vector v1 match or substantially match the positions of the characters and alphabets which are included as subjects in the target image I4 that is used as the reference image.

Fig. 7 is a diagram showing, by way of example, the target image I2 which is obtained in the target object imaging process S103 and the target image I2' which is obtained in the alignment process S105a. Positions of the characters and alphabets which are included as subjects in the target image I2 are shifted to the left relative to the positions of the characters and alphabets which are included as subjects in the target image I4 that is used as the reference image. Therefore, a displacement vector v2 points to the left as indicated by a white arrow in Fig. 7. Positions of the characters and alphabets which are included as subjects in the target image I2' obtained by shifting a pixel value of each pixel of the target image I2 by the displacement vector v2 match or substantially match the positions of the characters and alphabets which are included as subjects in the target image I4 used as the reference image.

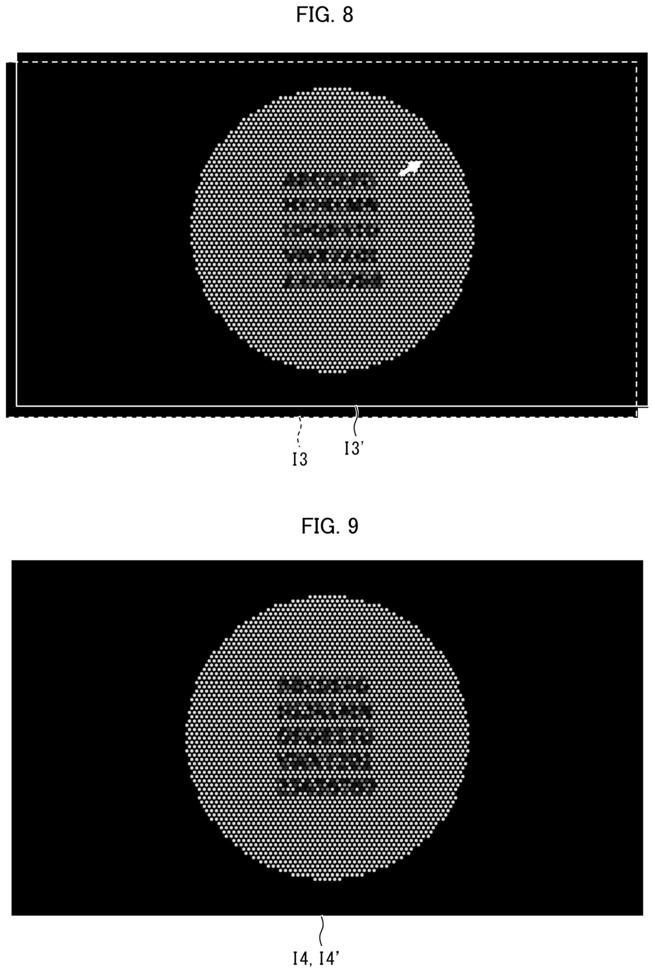

Fig. 8 is a diagram showing, by way of example, the target image I3 which is obtained in the target object imaging process S103 and the target image I3' which is obtained in the alignment process S105a. Positions of the characters and alphabets which are included as subjects in the target image I3 are shifted to the upper right relative to the positions of the characters and alphabets which are included as subjects in the target image I4 that is used as the reference image. Therefore, a displacement vector v3 points to the upper right as indicated by a white arrow in Fig. 8. Positions of the characters and alphabets which are included as subjects in the target image I3' obtained by shifting a pixel value of each pixel of the target image I3 by the displacement vector v3 match or substantially match the positions of the characters and alphabets which are included as subjects in the target image I4 used as the reference image.

Fig. 9 is a diagram showing, by way of example, the target image I4 which is obtained in the target object imaging process S103. Since the target image I4 is used as the reference image in the alignment process S105a, the alignment process S105a is not executed on the target image I4.

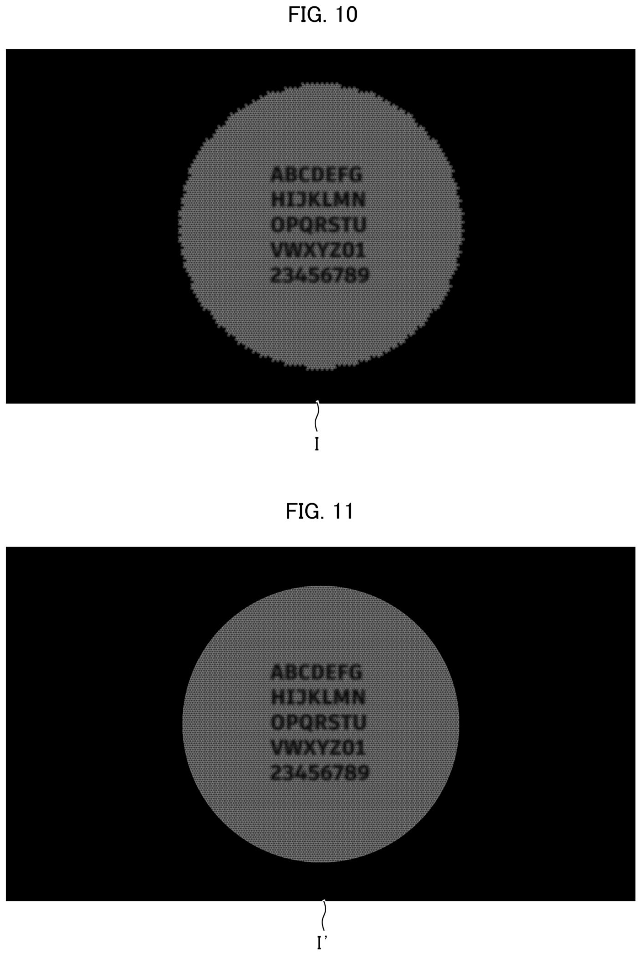

Fig. 10 is a diagram showing, by way of example, a super-resolution image I which is obtained in the super-resolution process S105. In the target images I1 to I4, it is difficult to read the alphabets and characters which are included as subjects. In contrast, in the super-resolution image I, it is easy to read the alphabets and characters which are included as subjects. In other words, it is confirmed that by performing the super-resolution process S105, the super-resolution image I having higher visibility than the target images I1 to I4 can be obtained.

Fig. 11 is a diagram showing, by way of example, an output image I' obtained in the mask process S106. In the super-resolution image I, jaggies (jaggedness caused by a configuration in which the image guide 13q is constituted by a plurality of cores) can be visually recognized at a boundary of the end face of the image guide 13q, the end face being included as an object. On the other hand, in the output image I', the jaggies included as objects cannot be visually recognized at the boundary of the end face of the image guide 13q. That is, it is confirmed that by performing the mask process S106, the output image I' having fewer jaggies than the super-resolution image I can be obtained.

### (Variation of image processing method)

The following description will discuss a variation of the image processing method S100, with reference to Fig. 12. Fig. 12 is a flowchart showing a flow of the image processing method S100'.

The image processing method S100' in accordance with the present variation is obtained by (1) adding a white standard plate imaging process S110, a mesh image acquisition process S111, a transparency process S112, and a core drawing process S113 to the above-described image processing method S100 and (2) replacing the averaging process S105b for taking a simple average in the above-described super-resolution process S105 by an averaging process S105c for taking a weighted average.

The white standard plate imaging process S110 and the mesh image acquisition process S111 are performed between the white balance adjustment process S102 and the target object imaging process S103 which are described above.

The white standard plate imaging process S110 is a process in which a user causes the objective lens 13r of the endoscope probe 13 to face a white standard plate, and the camera device 12 captures an image of the end face of the image guide 13q on the image guide plug part 13c side. The mesh image acquisition process S111 is a process in which the image processing device 10 acquires, from the camera device 12, an image captured in the white standard plate imaging process S110 and stores the image in the memory 102. The image captured in the white standard plate imaging process S101 and acquired in the mesh image acquisition process S111 is a color image in which a region corresponding to the cores of the image guide 13q is bright and other regions are dark. The image processing device 10 converts this color image to grayscale for use. A monochrome image obtained by converting this color image to grayscale is hereinafter referred to as a mesh image I0'. Note that a difference from the mesh image I0 described earlier is that the mesh image I0 is a color image which is captured before white balance adjustment, whereas the mesh image I0' is a monochrome image which is obtained by converting, to grayscale, a color image captured after white balance adjustment.

The transparency process S112 and the core drawing process S113 are pre-processes of the super-resolution process S105 which is performed between the target image acquisition process S104 and the super-resolution process S105 which are described above.

The transparency process S112 is a process in which, for each target image Ij (j is each natural number of 1 or more and n or less), the image processing device 10 refers to the mesh image I0' which is captured in the white standard plate imaging process S110, and causes the regions other than the region corresponding to the cores of the image guide 13q in the target image Ij (that is, regions corresponding to a mesh and a background in the mesh image I0') to be transparent or semi-transparent. An image generated by the transparency process S112 is a color image having opacity in which the opacity is high in the region corresponding to the cores of the image guide 13q and the opacity is low in the other regions. This image is hereinafter referred to as a "transparent target image Ij". A pixel value at a pixel (x, y) of the transparent target image Ij is obtained by, for example, adding a pixel value A(x, y) at a pixel (x, y) of the mesh image I0' as an opacity component to a pixel value {Rj(x, y), Gj(x, y), Bj(x, y)} at the pixel (x, y) of the target image Ij. That is, the pixel value at the pixel (x, y) of the transparent target image Ij is {Rj(x, y), Gj(x, y), Bj(x, y), A(x, y)}.

In the alignment process S105a of the super-resolution process S105, for each transparent target image Ii (i is each natural number of 1 or more and n-1 or less) other than the transparent target image In, the image processing device 10 (1) calculates a displacement vector vi of the transparent target image Ii with respect to the transparent target image In, and (2) shifts a pixel value of each pixel of the transparent target image Ii by the displacement vector vi. An image which is obtained by the alignment process S105a for the transparent target image Ii is a color image which has opacity and which represents an image of the target object O formed on the end face of the image guide 13q on the image guide plug part 13c side. This image is hereinafter referred to as a transparent target image Ii'. Note that the alignment process S105a is not performed for the transparent target image In which is acquired last. However, for convenience of description, the transparent target image In is hereinafter also referred to as a transparent target image In'. Specific examples of the transparent target images I1', I2', ... , In' which are obtained by the alignment process S105a will be described later with reference to different drawings.

In the averaging process S105c of the super-resolution process S105, the image processing device 10 generates the super-resolution image I by performing, for each component of each pixel (x, y), weighted averaging of pixel values {Rj'(x, y), Gj'(x, y), Bj'(x, y), Aj'(x, y)} of transparent target images Ij'. Opacity Aj'(x, y) is used as a weight in the weighted averaging. The image obtained in the averaging process S105c is a color image having opacity, which represents the image of the target object O formed on the end face of the image guide 13q on the image guide plug part 13c side. This image is hereinafter referred to as the super-resolution image I. A pixel value {R(x, y), G(x, y), B(x, y), A(x, y)} of the pixel (x, y) of the super-resolution image I is given by, for example, the formulas below. A specific example of the super-resolution image I generated in the averaging process S105c will be described later with reference to different drawings. $D \left(x, y\right) = A 1 ′ \left(x, y\right) + A 2 ′ \left(x, y\right) + … + An ′ \left(x, y\right) ;$
if $D \left(x, y\right) \neq 0 ,$ 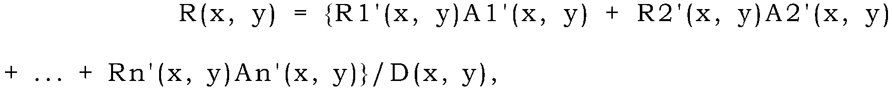 R(x, y) = {R1'(x, y)A1'(x, y) + R2'(x, y)A2'(x, y) + ... + Rn'(x, y)An'(x, y)}/D(x, y), 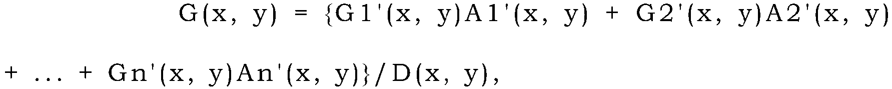 G(x, y) = {G1'(x, y)A1'(x, y) + G2'(x, y)A2'(x, y) + ... + Gn'(x, y)An'(x, y)}/D(x, y), 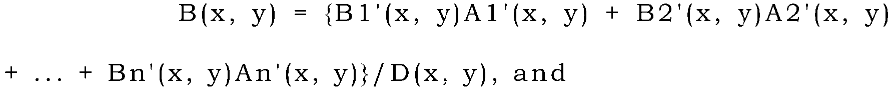 B(x, y) = {B1'(x, y)A1'(x, y) + B2'(x, y)A2'(x, y) + ... + Bn'(x, y)An'(x, y)}/D(x, y), and 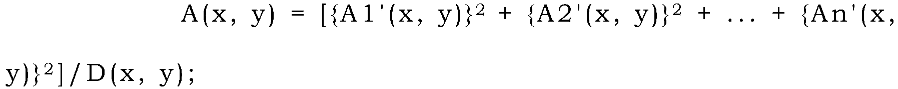 A(x, y) = [{A1'(x, y)}2 + {A2'(x, y)}2 + ... + {An'(x, y)}2]/D(x, y); and and
if $D \left(x, y\right) = 0 ,$ $R \left(x, y\right) = 0 ,$ $G \left(x, y\right) = 0 ,$ $B \left(x, y\right) = 0 ,$ and $A \left(x, y\right) = 0 .$

### (Execution example of image processing method in accordance with variation)

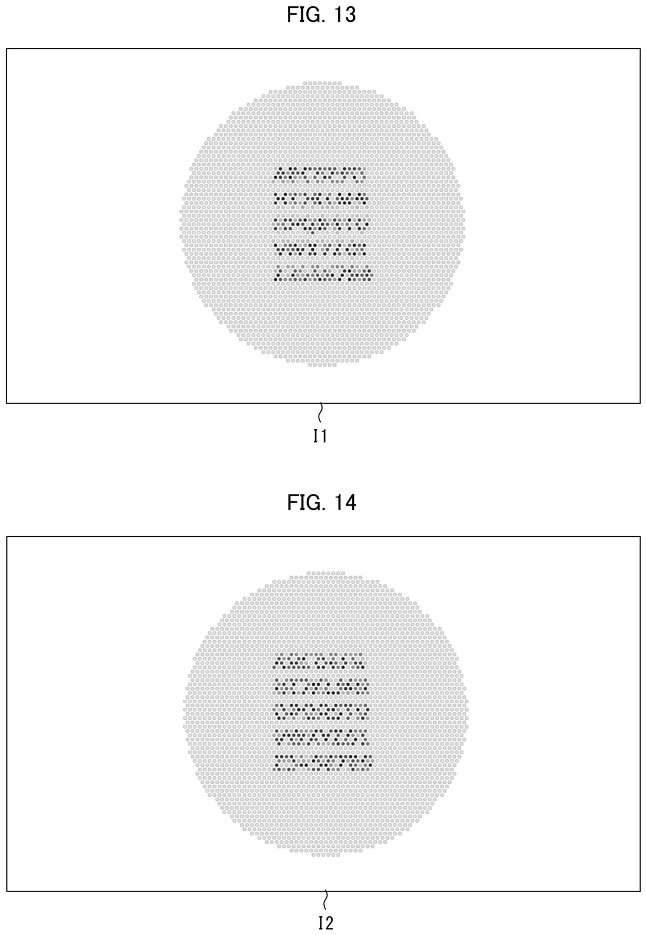

The following description will discuss an execution example of the image processing method S100 in accordance with the above-described variation with reference to Figs. 13 to 18. In this specific example, for simplicity, the number n of target images I1, I2, ... , In is set to 4.

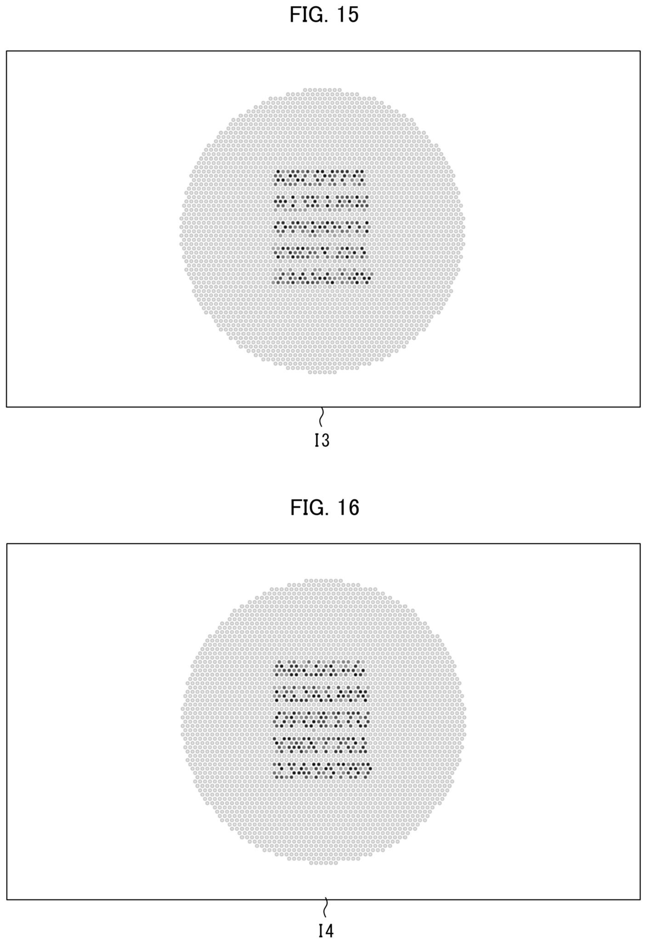

Fig. 13 is a diagram showing, by way of example, the transparent target image I1 which is obtained in the transparency process S112. Fig. 14 is a diagram showing, by way of example, the transparent target image I2 which is obtained in the transparency process S112. Fig. 15 is a diagram showing, by way of example, the transparent target image I3 which is obtained in the transparency process S112. Fig. 16 is a diagram showing, by way of example, the transparent target image I4 which is obtained in the transparency process S112. In any of the transparent target images Ii, it can be seen that the regions other than the region corresponding to the cores of the image guide 13q are made to be transparent.

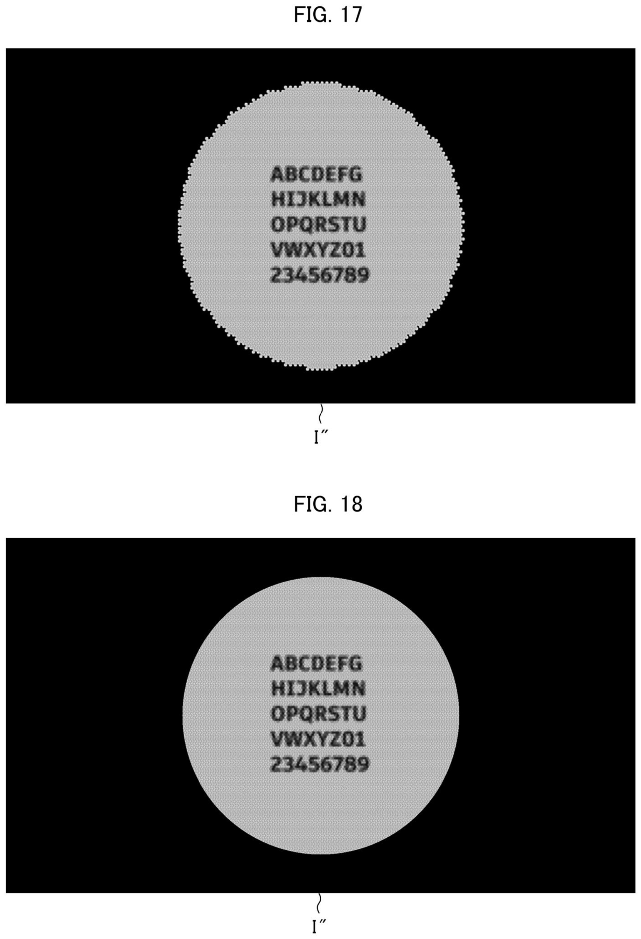

Fig. 17 is a diagram showing, by way of example, a super-resolution image I which is obtained in the super-resolution process S105. In the target images I1 to I4, it is difficult to read alphabets and characters included as subjects. In contrast, in the super-resolution image I, it is easy to read the alphabets and characters which are included as subjects. In other words, it is confirmed that by performing the super-resolution process S105, the super-resolution image I having higher visibility than the target images I1 to I4 can be obtained.

Further, from a comparison between the super-resolution image I (without the transparency process) shown in Fig. 10 and the super-resolution image I (with the transparency process) shown in Fig. 17, it is clear that the latter has higher contrast between the alphabets and characters and their background, and as a result, the latter has higher visibility of the alphabets and characters. This is for the following reason: in each target image Ii, since the regions other than the region corresponding to the cores of the image guide 13q have low brightness, a decrease in brightness and graininess of the image easily occur within a view circle in the super-resolution image I shown in Fig. 10; on the other hand, in each transparent target image Ii, since the regions other than the region corresponding to the cores of the image guide 13q are caused to be transparent, the decrease in brightness and the graininess of the image hardly occur within the view circle.

Note that the decrease in brightness and the graininess of the image occur because, for example, a pixel value of a pixel belonging to a low-brightness region of the target image Ij is averaged with a pixel value of a pixel belonging to a high-brightness region of another target image Ij', or a pixel value of a pixel belonging to a low-brightness region of a target image Ij is averaged with a pixel value of a pixel belonging to a low-brightness region of another target image I'.

Fig. 18 is a diagram showing, by way of example, an output image I' which is obtained in the mask process S106. In the super-resolution image I, jaggies (jaggedness caused by a configuration in which the image guide 13q is constituted by a plurality of cores) included as objects can be visually recognized at a boundary of the end face of the image guide 13q. On the other hand, in the output image I', the jaggies included as objects cannot be visually recognized at the boundary of the end face of the image guide 13q. That is, it is confirmed that by performing the mask process S106, the output image I' having fewer jaggies than the super-resolution image I can be obtained.

### (Further Variation 1 of image processing method)

As a pre-process of the above-described super-resolution process S105, the image processing device 10 may perform the core drawing process S113 on each transparent target image Ij.

In the core drawing process S113, the image processing device 10 first identifies a region corresponding to the cores of the image guide 13q (hereinafter, also referred to as a "core region") in a transparent target image Ij. For example, a set of pixels which have the same coordinates in the mesh image I0' and which have respective pixel values equal to or greater than a predetermined threshold is identified as the core region.

Next, for each of a red component, a green component, and a blue component, the image processing device 10 adjusts a pixel value of each of the pixels included in the core region in the transparent target image Ij so that: (a1) the pixel value matches a pixel value of a pixel located at the center of the core region (hereinafter, also referred to as a "center pixel value"); or (a2) the pixel value matches an average value of respective pixel values of the pixels included in the core region (hereinafter, also referred to as an "average pixel value"). Further, the image processing device 10 sets opacity of the pixel located at the center of the core region in the transparent target image Ij to 100%. Then, the image processing device 10 adjusts opacity of each pixel whose distance r from the center of the core region is equal to or less than a predetermined threshold value R in the transparent target image Ij so that: (b1) the opacity decreases linearly as the distance r increases; or (b2) the opacity decreases according to a Gaussian distribution as the distance r increases. Note that in a case where the opacity is not adjusted, the opacity of each pixel included in the core region matches the pixel value of the pixel having the same coordinates in the mesh image I0' as described above.
(a) of Fig. 19 is an enlarged view of the transparent target image Ij and the super-resolution image I which are obtained in a case where: the pixel value of each pixel included in the core region is adjusted to the average pixel value for the red component, the green component, and the blue component; but the opacity is not adjusted.
(b) of Fig. 19 is an enlarged view of the transparent target image Ij and the super-resolution image I which are obtained in a case where: the pixel value of each pixel included in the core region is adjusted to the average pixel value for the red component, the green component, and the blue component; and, with regard to the opacity, the opacity of each pixel whose distance r from the center of the core region is equal to or less than the predetermined threshold value R is adjusted so as to decrease linearly as the distance r increases. From a comparison between (a) and (b) of Fig. 19, it is clear that the decrease in brightness and the graininess of the image in the super-resolution image I can be further suppressed by adjusting the opacity. Note that a similar effect can be obtained also in a case where the pixel value of each of the pixels included in the core region is set to the center pixel value for each of the red component, the green component, and the blue component.
(c) of Fig. 19 is an enlarged view of the transparent target image Ij and the super-resolution image I which are obtained in a case where: the pixel value of each of the pixels included in the core region is adjusted to the average pixel value for the red component, the green component, and the blue component; and, with regard to the opacity, the opacity of each pixel whose distance r from the center of the core region is equal to or less than the predetermined threshold value R is adjusted so as to decrease according to a Gaussian distribution as the distance r increases. From a comparison between (a) and (c) of Fig. 19, it is clear that the decrease in brightness and the graininess of the image in the super-resolution image I can be further suppressed by adjusting the opacity. Furthermore, from a comparison between (b) and (c) of Fig. 19, it is clear that, from the viewpoint of suppressing the decrease in brightness and the graininess of the image in the super-resolution image I, adjusting the opacity such that the opacity decreases according to the Gaussian distribution as the distance r increases is more advantageous than adjusting the opacity such that the opacity decreases linearly as the distance r increases. Note that a similar effect can be obtained also in a case where the pixel value of each of the pixels included in the core region is set to the center pixel value for each of the red component, the green component, and the blue component.

### (Further Variation 2 of image processing method)

In the above-described super-resolution process S105, the image processing device 10 may perform the averaging process S105c with respect to all the transparent target images I1, I2, ... , In, or may perform the averaging process S105c with respect to some of the transparent target images I1, I2, ... , In.

As an example of the latter, a configuration is conceivable in which transparent target images other than a transparent target image having a large displacement amount among the transparent target images I1, I2, ... , In are subjected to the averaging process S105c. The transparent target image having a large displacement amount has little overlap with the transparent target image In that is used as the reference image in the alignment process S105a. Accordingly, a contribution to improvement in visibility of the super-resolution image I of such a transparent target image is limited. Further, adversely, the transparent target image may be a cause of generation of noise in the super-resolution image I. For this reason, by excluding the transparent target image having a large displacement amount from targets of the averaging process S105c, the generation of noise in the super-resolution image I can be suppressed without impairing the effect of improving the visibility of the super-resolution image I.

Note that examples of a method of selecting a transparent target image having a large displacement amount among the transparent target images I1, I2, ... , In include a method in which a transparent target image Ii having a displacement vector vi that exceeds a predetermined threshold value among the transparent target images I1, I2, ... , In is selected.

Alternatively, a configuration is conceivable in which transparent target images other than a transparent target image in which focus-related blur or motion-related blur has occurred, among the transparent target images I1, I2, ... , In, are subjected to the averaging process S105c. It is difficult to accurately perform the alignment process S105a for the transparent target image having significant focus-related blur or motion-related blur. In addition, since an outline of the target object O included as a subject is unclear, a contribution of such a transparent target image to improvement in visibility of the super-resolution image I is limited. Further, adversely, the transparent target image may be a cause of generation of noise in the super-resolution image I. For this reason, by excluding, from targets of the averaging process S105c, the transparent target image in which focus-related blur or motion-related blur has occurred, the generation of noise in the super-resolution image I can be suppressed without impairing the effect of improving the visibility of the super-resolution image I.

Note that examples of a method of selecting the transparent target image Ii in which focus-related blur or motion-related blur has occurred, among the transparent target images I1, I2, ... , In, include a method in which a transparent target image Ii in which differences |(vi - 1) - (vi)| and |(vi + 1) - (vi)| in displacement vectors from transparent target images Ii-1, Ii+1 immediately preceding and following the transparent target image Ii exceed a predetermined threshold value is selected. However, the method of selecting the transparent target image Ii in which focus-related blur or motion-related blur has occurred is not limited to this, and a known method can be arbitrarily employed.

### (Further Variation 3 of image processing method)

As a post-process of the above-described super-resolution process S105, the image processing device 10 may perform a base image generation process for generating a base image by smoothing a target image In which is obtained last, and an output image generation process for generating an output image by superimposing the super-resolution image I on the base image generated. A method of smoothing here is not particularly limited. The base image may be generated by smoothing using an averaging filter, or by smoothing using a Gaussian filter.

In a case where the opacity of a certain pixel is 0 in all the transparent target images I1', I2', ... , In', opacity of the image becomes 0 also in the super-resolution image I. That is, that pixel becomes transparent in the super-resolution image I. In a case where such a super-resolution image I is displayed, a background that is visible through the transparent pixel is visually recognized. This may give an unnatural impression. For example, when such a super-resolution image I is displayed in a case where a completely black background is used, the background that is visible through transparent pixels is visually recognized like scattered black spots. This gives an unnatural impression. Superimposing the super-resolution image I on the base image which has been generated by smoothing the target image In makes it possible to eliminate a concern that the background visible through the transparent pixels might be visually recognized and to display the super-resolution image I more naturally.

Note that the above description in the present variation has discussed a configuration in which the base image is generated by smoothing the target image In which is obtained last, but this should not be construed as a limitation. In other words, it is possible to employ a configuration in which the base image is generated by smoothing a target image Ii other than the target image In which is obtained last, among the target images I1, I2, ... , In.

### [Recap]

The present embodiment includes the following aspects.

### Aspect 1

An image processing method including: an acquisition process of acquiring a plurality of target images obtained by capturing an image of an end of an image guide, which has a plurality of cores and the other end of which faces a target object; and a super-resolution process of generating a super-resolution image by performing simple averaging or weighted averaging of pixel values of pixels corresponding to a same point of the target object in the plurality of target images.

This aspect makes it possible to obtain a super-resolution image having higher resolution or higher visibility than the target image.

### Aspect 2

The image processing method according to aspect 1, further including, as a pre-process of the super-resolution process, a transparency process of causing a region other than a region corresponding to the cores of the image guide to be transparent or semi-transparent in each of the plurality of target images,
the super-resolution process being a process in which the super-resolution image is generated by performing, with use of opacity as a weight, weighted averaging of pixel values of the pixels corresponding to the same point of the target object in the plurality of target images having undergone the pre-process.

This aspect makes it possible to obtain a super-resolution image having higher visibility by suppressing a decrease in brightness and graininess of the image either or both of which may occur in the super-resolution image.

### Aspect 3

The image processing method according to aspect 2, further including, as the pre-process, a core drawing process of setting a pixel value of each of pixels included in the region corresponding to the cores of the image guide in each of the plurality of target images, to a pixel value of a pixel located at a center of the region.

This aspect makes it possible to obtain a super-resolution image having higher visibility by further suppressing a decrease in brightness and graininess of the image either or both of which may occur in the super-resolution image.

### Aspect 4

The image processing method according to aspect 2, further including, as the pre-process, a core drawing process of setting a pixel value of each of pixels included in the region corresponding to the cores of the image guide in each of the plurality of target images, to an average value of respective pixel values of the pixels in the region.

This aspect makes it possible to obtain a super-resolution image having higher visibility by further suppressing a decrease in brightness and graininess of the image either or both of which may occur in the super-resolution image.

### Aspect 5

The image processing method according to aspect 3 or 4, wherein the core drawing process further includes setting the opacity of each of pixels whose distance from the center of the region is equal to or less than a predetermined threshold value in each of the plurality of target images so that, as the distance increases, the opacity decreases according to a Gaussian distribution.

This aspect makes it possible to obtain a super-resolution image having higher visibility by further suppressing a decrease in brightness and graininess of the image either or both of which may occur in the super-resolution image.

### Aspect 6

The image processing method according to any one of aspects 2 to 5, further including, as post-processes of the super-resolution process, a base image generation process of generating a base image by smoothing any one of the plurality of target images and a superimposition process of generating an output image by superimposing the super-resolution image on the base image.

This aspect makes it possible to obtain a more natural super-resolution image by eliminating the possibility that the background shows through in the super-resolution image.

### Aspect 7

The image processing method according to any one of aspects 2 to 6, wherein the super-resolution process is a process in which the super-resolution image is generated by performing weighted averaging of pixel values of pixels corresponding to a same point of the target object in target images whose displacement is determined to be small among the plurality of target images.

This aspect makes it possible to obtain a super-resolution image having higher visibility by reducing noise which may occur in the super-resolution image.

### Aspect 8

The image processing method according to any one of aspects 2 to 6, wherein the super-resolution process is a process in which the super-resolution image is generated by performing weighted averaging of pixel values of pixels corresponding to a same point of the target object in target images for which it is determined that no focus-related blur or no motion-related blur has occurred among the plurality of target images.

This aspect makes it possible to obtain a super-resolution image having higher visibility by reducing noise which may occur in the super-resolution image.

### Aspect 9

An image processing device including at least one processor, the processor being configured to execute: an acquisition process of acquiring a plurality of target images obtained by capturing an image of an end of an image guide, which has a plurality of cores and the other end of which faces a target object; and a super-resolution process of generating a super-resolution image by performing averaging or weighted averaging of pixel values of pixels corresponding to a same point of the target object in the plurality of target images.

This aspect makes it possible to obtain a super-resolution image having higher resolution or higher visibility than the target image.

### Aspect 10

The image processing device according to aspect 9, wherein: the processor is configured to further execute, as a pre-process of the super-resolution process, a transparency process of causing a region other than a region corresponding to the cores of the image guide to be transparent or semi-transparent in each of the plurality of target images; and the super-resolution process is a process in which the super-resolution image is generated by performing, with use of opacity as a weight, weighted averaging of pixel values of the pixels corresponding to the same point of the target object in the plurality of target images having undergone the pre-process.

This aspect makes it possible to obtain a super-resolution image having higher visibility by suppressing a decrease in brightness and graininess of the image either or both of which may occur in the super-resolution image.

### Aspect 11

The image processing device according to aspect 10, wherein the processor is configured to further execute, as the pre-process, a core drawing process of setting a pixel value of each of pixels included in the region corresponding to the cores of the image guide in each of the plurality of target images, to a pixel value of a pixel located at a center of the region.

This aspect makes it possible to obtain a super-resolution image having higher visibility by further suppressing a decrease in brightness and graininess of the image either or both of which may occur in the super-resolution image.

### Aspect 12

An image processing program for causing at least one processor to operate, the program causing the processor to execute:
an acquisition process of acquiring a plurality of target images obtained by capturing an image of an end of an image guide, which has a plurality of cores and the other end of which faces a target object; and a super-resolution process of generating a super-resolution image by performing averaging or weighted averaging of pixel values of pixels corresponding to a same point of the target object in the plurality of target images.

This aspect makes it possible to obtain a super-resolution image having higher resolution or higher visibility than the target image.

### Aspect 13

The image processing program according to aspect 12, wherein: the program causes the processor to further execute, as a pre-process of the super-resolution process, a transparency process of causing a region other than a region corresponding to the cores of the image guide to be transparent or semi-transparent in each of the plurality of target images; and the super-resolution process is a process in which the super-resolution image is generated by performing, with use of opacity as a weight, weighted averaging of pixel values of the pixels corresponding to the same point of the target object in the plurality of target images having undergone the pre-process.

This aspect makes it possible to obtain a super-resolution image having higher visibility by further suppressing a decrease in brightness and graininess of the image either or both of which may occur in the super-resolution image.

### Aspect 14

The image processing program according to aspect 13, wherein the program causes the processor to further execute, as the pre-process, a core drawing process of setting a pixel value of each of pixels included in the region corresponding to the cores of the image guide in each of the plurality of target images, to a pixel value of a pixel located at the center of the region.

This aspect makes it possible to obtain a super-resolution image having higher visibility by further suppressing a decrease in brightness and graininess of the image either or both of which may occur in the super-resolution image.

### Aspect 15

An endoscope system including: an image processing device described in any one of aspects 9 to 11; and an endoscope probe including the image guide.

This aspect can realize an endoscope system which makes it possible to obtain a super-resolution image having higher resolution or higher visibility than the target image.

### (Additional Remarks)

The present invention is not limited to the embodiments described above, but may be altered in various ways by a skilled person within the scope of the claims. Any embodiment based on a proper combination of technical means disclosed in the embodiments is also encompassed in the technical scope of the present invention.

### Reference Signs List

1 endoscope system
10 image processing device
101 processor
102 memory
103 storage
104 input/output interface
105 bus
11 light source device
12 camera device
13 endoscope probe
13p light guide
13q image guide

## Claims

1. An image processing method comprising:
an acquisition process of acquiring a plurality of target images obtained by capturing an image of an end of an image guide, which has a plurality of cores and the other end of which faces a target object; and
a super-resolution process of generating a super-resolution image by performing simple averaging or weighted averaging of pixel values of pixels corresponding to a same point of the target object in the plurality of target images.

2. The image processing method according to claim 1, further comprising, as a pre-process of the super-resolution process, a transparency process of causing a region other than a region corresponding to the cores of the image guide to be transparent or semi-transparent in each of the plurality of target images,
the super-resolution process being a process in which the super-resolution image is generated by performing, with use of opacity as a weight, weighted averaging of pixel values of the pixels corresponding to the same point of the target object in the plurality of target images having undergone the pre-process.

3. The image processing method according to claim 2, further comprising, as the pre-process, a core drawing process of setting a pixel value of each of pixels included in the region corresponding to the cores of the image guide in each of the plurality of target images, to a pixel value of a pixel located at a center of the region.

4. The image processing method according to claim 2, further comprising, as the pre-process, a core drawing process of setting a pixel value of each of pixels included in the region corresponding to the cores of the image guide in each of the plurality of target images, to an average value of respective pixel values of the pixels in the region.

5. The image processing method according to claim 3 or 4, wherein
the core drawing process further includes setting the opacity of each of pixels whose distance from the center of the region is equal to or less than a predetermined threshold value in each of the plurality of target images so that, as the distance increases, the opacity decreases according to a Gaussian distribution.

6. The image processing method according to any one of claims 2 to 4, further comprising, as post-processes of the super-resolution process, a base image generation process of generating a base image by smoothing any one of the plurality of target images and a superimposition process of generating an output image by superimposing the super-resolution image on the base image.

7. The image processing method according to any one of claims 2 to 4, wherein
the super-resolution process is a process in which the super-resolution image is generated by performing weighted averaging of pixel values of pixels corresponding to a same point of the target object in target images whose displacement is determined to be small among the plurality of target images.

8. The image processing method according to any one of claims 2 to 4, wherein
the super-resolution process is a process in which the super-resolution image is generated by performing weighted averaging of pixel values of pixels corresponding to a same point of the target object in target images for which it is determined that no focus-related blur or no motion-related blur has occurred among the plurality of target images.

9. An image processing device comprising at least one processor,
the processor being configured to execute:
an acquisition process of acquiring a plurality of target images obtained by capturing an image of an end of an image guide, which has a plurality of cores and the other end of which faces a target object; and
a super-resolution process of generating a super-resolution image by performing averaging or weighted averaging of pixel values of pixels corresponding to a same point of the target object in the plurality of target images.

10. The image processing device according to claim 9, wherein:
the processor is configured to further execute, as a pre-process of the super-resolution process, a transparency process of causing a region other than a region corresponding to the cores of the image guide to be transparent or semi-transparent in each of the plurality of target images; and
the super-resolution process is a process in which the super-resolution image is generated by performing, with use of opacity as a weight, weighted averaging of pixel values of the pixels corresponding to the same point of the target object in the plurality of target images having undergone the pre-process.

11. The image processing device according to claim 10, wherein the processor is configured to further execute, as the pre-process, a core drawing process of setting a pixel value of each of pixels included in the region corresponding to the cores of the image guide in each of the plurality of target images, to a pixel value of a pixel located at a center of the region.

12. An image processing program for causing at least one processor to operate, the program causing the processor to execute:
an acquisition process of acquiring a plurality of target images obtained by capturing an image of an end of an image guide, which has a plurality of cores and the other end of which faces a target object; and
a super-resolution process of generating a super-resolution image by performing averaging or weighted averaging of pixel values of pixels corresponding to a same point of the target object in the plurality of target images.

13. The image processing program according to claim 12, wherein:
the program causes the processor to further execute, as a pre-process of the super-resolution process, a transparency process of causing a region other than a region corresponding to the cores of the image guide to be transparent or semi-transparent in each of the plurality of target images; and
the super-resolution process is a process in which the super-resolution image is generated by performing, with use of opacity as a weight, weighted averaging of pixel values of the pixels corresponding to the same point of the target object in the plurality of target images having undergone the pre-process.

14. The image processing program according to claim 13, wherein the program causes the processor to further execute, as the pre-process, a core drawing process of setting a pixel value of each of pixels included in the region corresponding to the cores of the image guide in each of the plurality of target images, to a pixel value of a pixel located at the center of the region.

15. An endoscope system comprising:
an image processing device recited in any one of claims 9 to 11; and
an endoscope probe including the image guide.
